# EUROPÄISCHE PATENTSCHRIFT

(11) **EP 2 138 570 B1**
(45) Veröffentlichungstag und Bekanntmachung des Hinweises auf die Patenterteilung: **19.05.2010**
(21) Anmeldenummer: 09007735.5
(22) Anmeldetag: 12.06.2009
(51) Int. Cl.: C12M 1/12

(54) **Vorrichtung und Verfahren zum koninuierlichen Abführen von partikelarmer Lösung aus einem Bioreaktor**
Device and method for continuously removing a low-particle solution from a bioreactor
Dispositif et procédé d'évacuation continue de solution pauvre en particules d'un bioréacteur

(30) Priorität: 25.06.2008 DE 102008029836
(43) Veröffentlichungstag der Anmeldung: 30.12.2009
(73) Patentinhaber: Sartorius Stedim Biotech GmbH, 37079 Göttingen (DE)
(72) Erfinder: Tappe, Alexander Dr., 37081 Göttingen (DE)

(56) Entgegenhaltungen:
- EP-A- 0 164 608
- CH-A5- 669 783
- DE-A1- 10 022 635
- DE-A1- 19 507 456
- DE-T2- 69 325 163
- US-A1- 2007 241 041

## Beschreibung

### Gebiet der Erfindung

Die Erfindung betrifft eine Vorrichtung zum kontinuierlichen Abführen von partikelarmer Lösung aus einem Bioreaktor mittels Partikeltrennung in einem Hydrozyklon, wobei der Bioreaktor über eine Feedleitung mit einer zwischengeschalteten ersten Pumpe mit einem Zulauf des Hydrozyklons und über eine Rückleitung mit einem Unterlauf des Hydrozyklons verbunden ist, wobei die partikelarme Lösung über einen Überlauf des Hydrozyklons abführbar ist.

Die Erfindung betrifft weiterhin ein Verfahren zum kontinuierlichen Abführen von partikelarmer Lösung aus einem Bioreaktor mittels Partikeltrennung in einem Hydrozyklon, wobei aus dem Bioreaktor über eine Feedleitung mit einer zwischengeschalteten ersten Pumpe partikelhaltige Lösung entnommen, dem Hydrozyklon über einen Zulauf zugeführt und in einen partikelarmen Overflow und einen partikelreicheren Underflow aufgeteilt wird, wobei der Underflow über eine Rückleitung in den Bioreaktor rückführbar ist und der Overflow abgeführt wird.

### Stand der Technik

Aus der DE 100 22 635 A1 sind eine Vorrichtung und ein Verfahren zum kontinuierlichen Abführen von zellarmer Lösung aus einem Bioreaktor bekannt, der in einem Kreislauf mit einem Hydrozyklon verbunden ist. Dabei wird dem Bioreaktor zellhaltige Lösung bzw. Zellsuspension entnommen und über eine Feedleitung dem Zulauf des Hydrozyklons zugeführt, der die zellhaltige Lösung in einen zellarmen Overflow und in einem mit Zellen angereicherten Underflow aufteilt. Der Underflow wird über eine Rückleitung dem Bioreaktor wieder zugeführt, während der Overflow abgeführt wird.

Nachteilig bei einer derartigen Perfusionsfahrweise des Bioreaktors ist, dass aufgrund des geringen Dichteunterschieds zwischen Tierzellen und Medien der Hydrozyklon mit hohen Flussraten betrieben werden muss, was zu hohen Overflowraten führt. Weiterhin nachteilig ist, dass die Trennleistung von Hydrozyklonen durch Schwankungen der Feedgeschwindigkeit stark beeinträchtigt wird. So ist es beispielsweise nicht möglich, mit einem 2,5 Milliliter Hydrozyklon einen 200 Liter Bioreaktor kontinuierlich zu betreiben.

Ist beispielsweise die zur Abtrennung benötigte Feedrate sehr groß gegenüber der gewünschten Verdünnungsrate des Bioreaktors und wird der Hydrozyklon bei der gewünschten Verdünnungsrate betrieben, kann der Hydrozyklon die Zellen nicht abtrennen.

Wird andererseits der Hydrozyklon bei einer dem Trennproblem geeigneten Feedrate betrieben, werden zu viel Medium und zu viele Zellen aus dem Reaktor entfernt. Dies führt zu unerwünscht hohen Materialkosten für das Medium bzw. die Lösung und zu geringen Produktkonzentrationen im Overflow, was die weitere Aufreinigung des Produktes schwieriger macht. So definiert bei den bekannten Vorrichtungen der Dichteunterschied die Geometrie, d.h. die Mindestdurchmesser der Zu- und Abgänge des Hydrozyklons sowie die Mindestgröße seines Innenvolumens. Der Hydrozyklon kann daher praktisch nicht für kleinere Bioreaktoren ausgelegt werden.

Weiterhin ist es aus der EP 0 164 608 A2 bekannt, einen Hydrozyklon mit einem Bioreaktor in einen Kreislauf anzuordnen. Dabei wird eine Biosuspension aus einem Bioreaktor dem Einlass eines Hydrozyklons zugeführt und in ein weitgehend zellfreies Medium und ein zellangereichertes Medium aufgeteilt. Das zellangereicherte Medium wird über einen Auslass in den Bioreaktor zurückgeführt. Insbesondere bei weitgehend gleichen Molekulargewichten des Produkts und Bestandteilen der Nährlösung wird das weitgehend zellfreie Medium einer im Hydrozyklon angeordneten Filtriereinrichtung zugeführt, die beispielsweise als eine Pervaporationsmembran mit einer vorgelagerten Porenmembran ausgebildet ist.

Grundsätzlich weist auch diese bekannte Vorrichtung die oben beschriebenen Nachteile auf.

### Aufgabenstellung

Aufgabe der vorliegenden Erfindung ist es daher, die bekannten Vorrichtungen und Verfahren so zu verbessern, dass der Hydrozyklon auch an Bioreaktoren mit kleinen Verdünnungsraten mit hoher Effizienz betrieben werden kann. Weiterhin soll eine Beeinträchtigung der Trennleistung von Hydrozyklonen durch Schwankungen der Feedgeschwindigkeit vermieden werden.

### Darstellung der Erfindung

Die Aufgabe bezüglich der Vorrichtung wird in Verbindung mit dem Oberbegriff des Anspruches 1 dadurch gelöst, dass zwischen Unterlauf und Zulauf des Hydrozyklons eine Umlaufleitung mit einer zweiten Pumpe angeordnet ist.

Über die zweite Pumpe kann ein Teil des Underflows direkt in den Feed, also dem Hydrozyklon über den Zulauf wieder zugeführt werden, während beispielsweise nur ein kleiner Teil in den Reaktor zurückgeführt wird. Damit kann praktisch unabhängig von der Verdünnungsrate des Bioreaktors der für die einwandfreie Funktion des Hydrozyklons benötigte Druckabfall aufgebaut werden. Dadurch kann der Hydrozyklon an Bioreaktoren mit kleinen Verdünnungsraten mit hoher Effizienz betrieben werden.

Gemäß einer bevorzugten Ausführungsform der Erfindung ist eine Steuereinheit vorgesehen, über die die Pumpen steuerbar sind. Hierbei ist über die zweite Pumpe der Druckabfall des Hydrozyklons bestimmbar während über die erste Pumpe die Verdünnungsrate des Bioreaktors bestimmbar ist.

Nach einer weiteren bevorzugten Ausführungsform der Erfindung ist am Hydrozyklon ein zweiter mit der Feedleitung verbundener Zulauf angeordnet.

Durch den zweiten Zulauf ist eine bessere Verteilung des eingebrachten Mediums bzw. der eingebrachten Lösung im Hydrozyklon möglich.

Gemäß einer bevorzugten Ausführungsform der Erfindung weist der Hydrozyklon im Bereich des Überlaufs, ggf. auch innerhalb des Hydrozyklons, einen Filter auf.

So können zum einen Restpartikel zurückgehalten und zum anderen können Stoffe mit gleichem Molekulargewicht des abzutrennenden Produktes herausgefiltert werden.

Nach einer weiteren bevorzugten Ausführungsform der Erfindung sind der Bioreaktor, der Hydrozyklon, die Pumpen bzw. Pumpenköpfe und die entsprechenden Verbindungsleitungen als Einwegteile ausgebildet und können als Einheit steril verpackt geliefert werden.

Die das Verfahren betreffende Aufgabe wird in Verbindung mit dem Oberbegriff des Anspruchs 8 dadurch gelöst, dass ein erster Teil des Underflows über eine Umlaufleitung mit dazwischengeschalteter zweiter Pumpe dem Zulauf des Hydrozyklons wieder zugeführt wird, während der andere zweite Teil des Underflows in den Bioreaktor zurückgeführt wird.

Dadurch, dass ein erster Teil des Underflows über eine Umlaufleitung mit dazwischengeschalteter zweiter Pumpe dem Zulauf des Hydrozyklons wieder zugeführt wird, während der andere zweite Teil des Underflows in den Bioreaktor zurückgeführt wird, können vorteilhafterweise die Verdünnungsrate des Bioreaktors und der am Hydrozyklon benötigte Druckabfall bzw. Druckdifferenz getrennt voneinander geregelt werden, sodass der Hydrozyklon auch für kleine Bioreaktoren verwendet werden kann. Der Hydrozyklon kann auch an Bioreaktoren mit kleinen Verdünnungsraten mit hoher Effizienz betrieben werden.

Die im Hydrozyklon benötigte Druckdifferenz kann somit über die zweite Pumpe und die Verdünnungsrate des Bioreaktors über die erste Pumpe geregelt werden.

Der zellarme Overflow kann zusätzlich einem Filter zugeführt und gefiltert werden.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:

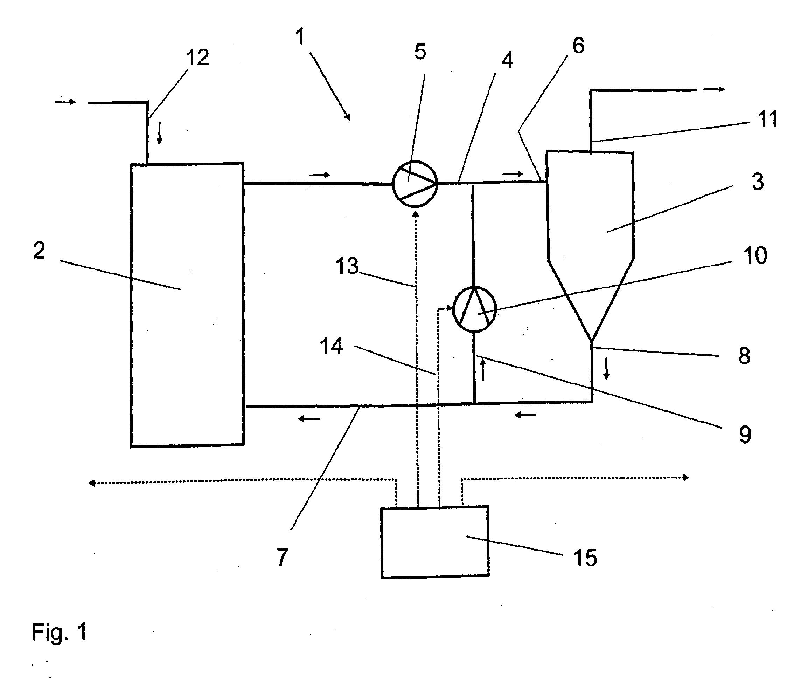

- Figur 1:: eine schematische Darstellung einer Vorrichtung zum kontinuierlichen Abführen von partikelarmer Lösung mit in einem Kreislauf angeordnetem Bioreaktor und Hydrozyklon,
- Figur 2:: eine schematische Darstellung einer weiteren Vorrichtung zum kontinuierlichen Abführen von partikelarmer Lösung bei der am Hydrozyklon ein zweiter mit einer Feedleitung verbundener Zulauf angeordnet ist und
- Figur 3:: eine schematische Darstellung einer weiteren Vorrichtung bei der der Hydrozyklon einen zweiten Zulauf aufweist, der über eine zweite Pumpe mit dem Unterlauf verbunden ist.

### Beschreibung der Ausführungsbeispiele

Eine Vorrichtung 1 zum kontinuierlichen Abführen von partikelarmer Lösung besteht im Wesentlichen aus einem Bioreaktor 2 und einem Hydrozyklon 3, die in einem Kreislauf miteinander verbunden sind.

Der Bioreaktor 2 ist über eine Feedleitung 4 und einer zwischengeschalteten ersten Pumpe 5 mit einem Zulauf 6 des Hydrozyklons 2 verbunden. Über eine Rückleitung 7 ist ein Unterlauf 8 des Hydrozyklons 3 mit dem Bioreaktor 2 verbunden.

Zwischen Unterlauf 8 und Zulauf 6 ist eine Umlaufleitung 9 mit einer zweiten Pumpe 10 angeordnet. In vertikaler Richtung oben weist der Hydrozyklon einen Überlauf 11 auf, über den Medium abgeführt werden kann. Dem Bioreaktor 2 kann über eine Zulaufleitung 12 frisches Medium zugeführt werden.

Die Pumpen 5, 10 sind über Steuerleitungen 13, 14 mit einer sie steuernden Steuereinheit 15 verbunden.

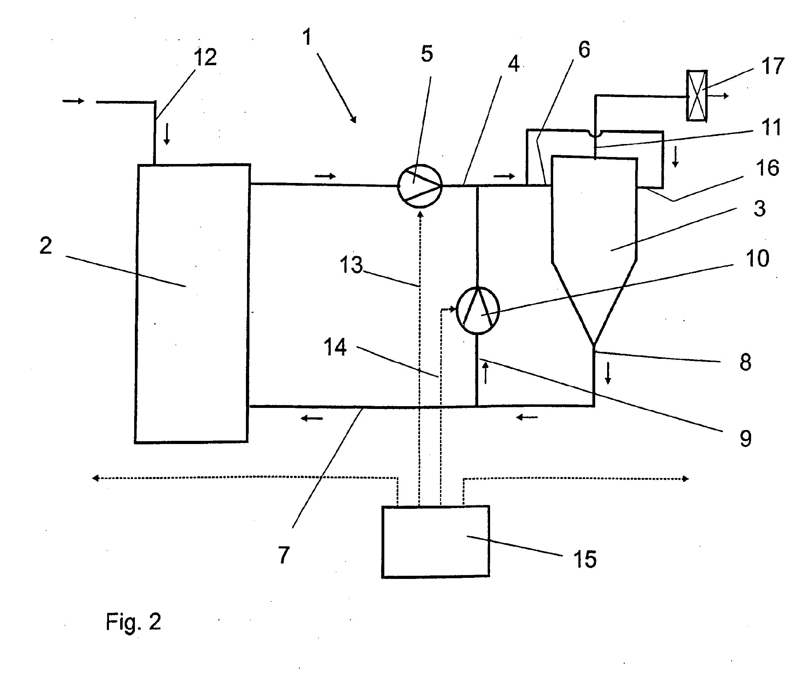

Gemäß dem Ausführungsbeispiel von Figur 2 ist am Hydrozyklon 3 ein zweiter Zulauf 16 angeordnet, der mit der Feedleitung 4 verbunden ist. Der Überlauf 11 von Figur 2 ist zudem mit einem Filter 17 verbunden.

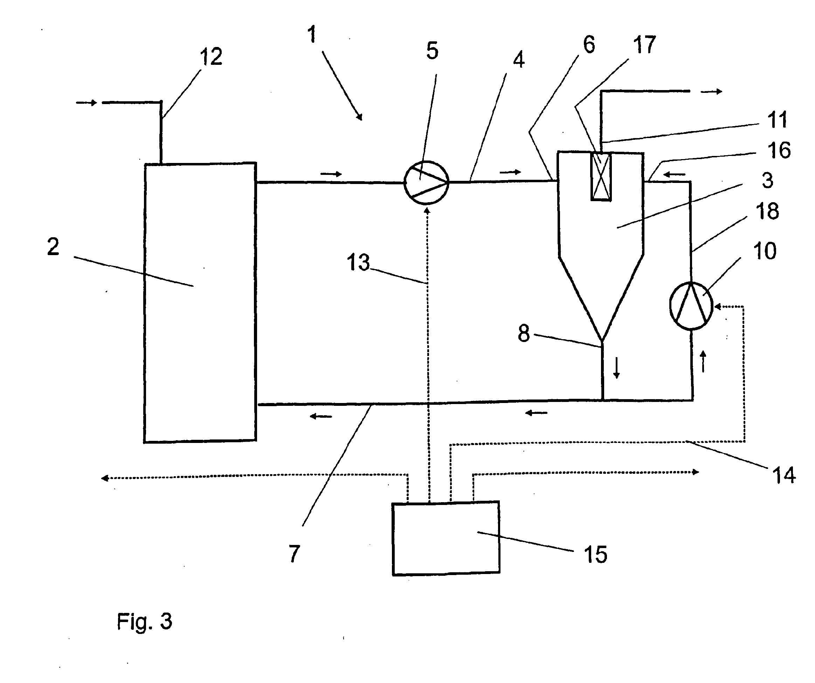

Entsprechend dem Ausführungsbeispiel der Figur 3 ist der Unterlauf 8 über eine Umlaufleitung 18 mit dem zweiten Zulauf 16 verbunden. Figur 3 zeigt weiterhin einen Filter 17, der im Hydrozyklon 3 angeordnet ist und in den Überlauf 11 mündet.

Aus dem als Perfusionsreaktor betriebenen Bioreaktor 2 wird über die Feedleitung 4 partikel- bzw. zellhaltige Lösung entnommen und dem Hydrozyklon 3 über den Zulauf 6 bzw. zweiten Zulauf 16 zugeführt und von dem Hydrozyklon 3 in einen partikel- bzw. zellarmen Overflow und einen partikelreichen bzw. zellreichen Underflow aufgeteilt, wobei der Underflow über die Rückleitung 7 in den Bioreaktor 2 zurückgeführt wird. Der partikel- bzw. zellarme Overflow wird über den Überlauf 11 abgeführt. Dabei kann der Overflow noch in dem Filter 17 gefiltert werden.

Je nach Einstellung wird ein erster Teil des Underflows über die Umlaufleitung 9 mit der dazwischengeschalteten zweiten Pumpe 10 dem Zulauf 6 bzw. zweiten Zulauf 16 des Hydrozyklons 3 wieder zugeführt, während der andere zweite Teil des Underflows über die Rückleitung 7 in den Bioreaktor 2 zurückgeführt wird.

Natürlich stellen die in der speziellen Beschreibung diskutierten und in den Figuren gezeigten Ausführungsformen nur illustrative Ausführungsbeispiele der vorliegenden Erfindung dar.

## Patentansprüche

1. Vorrichtung zum kontinuierlichen Abführen von partikelarmer Lösung aus einem Bioreaktor (2) mittels Partikeltrennung in einem Hydrozyklon (3), wobei der Bioreaktor (2) über eine Feedleitung (4) mit einer zwischengeschalteten ersten Pumpe (5) mit einem Zulauf (6) des Hydrozyklons (3) und über eine Rückleitung (7) mit einem Unterlauf (8) des Hydrozyklons (3) verbunden ist, wobei die partikelarme Lösung über einen Überlauf (11) des Hydrozyklons (3) abführbar ist,
**dadurch gekennzeichnet,**
**dass** zwischen Unterlauf (8) und Zulauf (6,16) des Hydrozyklons (3) eine Umlaufleitung (9, 18) mit einer zweiten Pumpe (10) angeordnet ist.

2. Vorrichtung nach Anspruch 1,
**dadurch gekennzeichnet,**
**dass** eine Steuereinheit (15) vorgesehen ist, über die die Pumpen (5,10) steuerbar sind.

3. Vorrichtung nach Anspruch 1 oder 2,
**dadurch gekennzeichnet,**
**dass** über die zweite Pumpe (10) der Druckabfall des Hydrozyklons (3) bestimmbar ist.

4. Vorrichtung nach einem der Ansprüche 1 bis 3,
**dadurch gekennzeichnet,**
**dass** über die erste Pumpe (5) die Verdünnungsrate des Bioreaktors (2) bestimmbar ist.

5. Vorrichtung nach einem der Ansprüche 1 bis 4,
**dadurch gekennzeichnet,**
**dass** am Hydrozyklon (3) ein zweiter mit der Feedleitung (4) verbundener Zulauf (16) angeordnet ist.

6. Vorrichtung nach einem der Ansprüche 1 bis 5,
**dadurch gekennzeichnet,**
**dass** der Hydrozyklon (3) im Bereich des Überlaufs (11) einen Filter (17) aufweist.

7. Vorrichtung nach einem der Ansprüche 1 bis 6,
**dadurch gekennzeichnet,**
**dass** der Bioreaktor (2) und/oder der Hydrozyklon (3) und/oder die Pumpen (5,10) oder ihre Pumpenköpfe und/oder die Leitungen (4,7,9,12,18) als Einwegteile ausgebildet sind.

8. Verfahren zum kontinuierlichen Abführen von partikelarmer Lösung aus einem Bioreaktor (2) mittels Partikeltrennung in einem Hydrozyklon (3), wobei aus dem Bioreaktor (2) über eine Feedleitung (4) mit einer zwischengeschalteten ersten Pumpe (5) partikelhaltige Lösung entnommen, dem Hydrozyklon (3) über einen Zulauf (6, 16) zugeführt und in einen partikelarmen Overflow und einen partikelreicheren Underflow aufgeteilt wird, wobei der Underflow über eine Rückleitung (7) in den Bioreaktor (2) rückführbar ist und der Overflow abgeführt wird, **dadurch gekennzeichnet,**
**dass** ein erster Teil des Underflows über eine Umlaufleitung (9, 18) mit dazwischengeschalteter zweiter Pumpe (10) dem Zulauf (6, 16) des Hydrozyklons (3) wieder zugeführt wird, während der andere zweite Teil des Underflows in den Bioreaktor (2) zurückgeführt wird.

9. Verfahren nach Anspruch 8,
**dadurch gekennzeichnet,**
**dass** aus dem Bioreaktor (2) ein zellhaltiges Medium in den Hydrozyklon (3) als Lösung gefördert und in einen zellarmen Overflow und einen zellreichen Underflow aufgeteilt wird.

10. Verfahren nach Anspruch 8 oder 9,
**dadurch gekennzeichnet,**
**dass** eine im Hydrozyklon (3) benötigte Druckdifferenz über die zweite (10) Pumpe geregelt wird.

11. Verfahren nach einem der Ansprüche 8 bis 10,
**dadurch gekennzeichnet,**
**dass** die Verdünnungsrate des Bioreaktors (2) über die erste Pumpe (5) geregelt wird.

12. Verfahren nach einem der Ansprüche 8 bis 11,
**dadurch gekennzeichnet,**
**dass** der zellarme Overflow einem Filter (17) zugeführt und gefiltert wird.

## Claims

1. Device for continuous removal of low-particle solution from a bioreactor (2) by means of particle separation in a hydrocyclone (3), wherein the bioreactor (2) is connected with an inlet (6) of the hydrocyclone (3) by way of a feed duct (4) with a intermediate first pump (5) and by way of a return duct (7) with an underneath outlet (8) of the hydrocyclone (3), wherein the low-particle solution is removable by way of an overhead outlet (11) of the hydrocyclone (3), **characterised in that** a circulating duct (9, 18) with a second pump (10) is arranged between underneath outlet (8) and inlet (6, 16) of the hydrocyclone (3).

2. Device according to claim 1, **characterised in that** a control unit (15) by way of which the pumps (5, 10) are controllable is provided.

3. Device according to claim 1 or 2, **characterised in that** the pressure drop of the hydrocyclone (3) is determinable by way of the second pump (10).

4. Device according to any one of claims 1 to 3, **characterised in that** the dilution rate of the bioreactor (2) is determinable by way of the first pump (5).

5. Device according to any one of claims 1 to 4, **characterised in that** a second inlet (16) connected with the feed duct (4) is arranged at the hydrocyclone (3).

6. Device according to any one of claims 1 to 5, **characterised in that** the hydrocyclone (3) has a filter (17) in the region of the overhead outlet (11).

7. Device according to any one of claims 1 to 6, **characterised in that** the bioreactor (2) and/or the hydrocyclone (3) and/or the pumps (5, 10) or the pump heads thereof and/or the ducts (4, 7, 9, 12, 18) is or are constructed as single-use components.

8. Method for continuous removal of low-particle solution from a bioreactor (2) by means of particle separation in a hydrocyclone (3), wherein solution with particle content is removed from the bioreactor (2) by way of a feed duct (4) with an intermediate first pump (5), fed to the hydrocyclone (3) by way of an inlet (6, 16) and divided up into a low-particle overflow and particle-richer underflow, wherein the underflow is returnable to the bioreactor (2) by way of a return duct (7) and the overflow is conducted away, **characterised in that** a first part of the underflow is fed back to the inlet (6, 16) of the hydrocyclone (3) by way of a circulating duct (9, 18) with intermediate second pump (10), whilst the other, second part of the underflow is led back to the bioreactor (2).

9. Method according to claim 8, **characterised in that** a cell-containing medium is conveyed from the bioreactor (2) into the hydrocyclone (3) as solution and is divided up into a low-cell overflow and a cell-rich underflow.

10. Method according to claim 8 or 9, **characterised in that** a pressure difference required in the hydrocyclone (3) is regulated by way of the second pump (10).

11. Method according to any one of claims 8 to 10, **characterised in that** the dilution rate of the bioreactor (2) is regulated by way of the first pump (5).

12. Method according to any one of claims 8 to 11, **characterised in that** the low-cell overflow is fed to a filter (17) and filtered.

## Revendications

1. Dispositif pour l'évacuation continue d'une solution pauvre en particules hors d'un bioréacteur (2) au moyen d'une séparation de particules dans un hydrocyclone (3), le bioréacteur (2) étant relié à une admission (6) de l'hydrocyclone (3) via une conduite d'alimentation (4) contenant une première pompe (5) intercalée et à une sortie inférieure (8) de l'hydrocyclone (3) via une conduite de retour (7), la solution pauvre en particules pouvant être évacuée via une sortie supérieure (11) de l'hydrocyclone (3), **caractérisé en ce qu'**une conduite de dérivation (9, 18) contenant une deuxième pompe (10) est montée entre la sortie inférieure (8) et l'admission (6, 16) de l'hydrocyclone (3).

2. Dispositif selon la revendication 1, **caractérisé en ce qu'**il est prévu une unité de commande (15), par l'intermédiaire de laquelle les pompes (5, 10) peuvent être commandées.

3. Dispositif selon la revendication 1 ou 2, **caractérisé en ce que** la chute de pression de l'hydrocyclone (3) peut être déterminée par l'intermédiaire de la deuxième pompe (10).

4. Dispositif selon l'une quelconque des revendications 1 à 3, **caractérisé en ce que** le taux de dilution du bioréacteur (2) peut être déterminé par l'intermédiaire de la première pompe (5).

5. Dispositif selon l'une quelconque des revendications 1 à 4, **caractérisé en ce qu'**une deuxième admission (16), reliée à la conduite d'alimentation (4), est disposée sur l'hydrocyclone (3).

6. Dispositif selon l'une quelconque des revendications 1 à 5, **caractérisé en ce que** l'hydrocyclone (3) comporte un filtre (17) dans la zone de la sortie supérieure (11).

7. Dispositif selon l'une quelconque des revendications 1 à 6, **caractérisé en ce que** le bioréacteur (2) et/ou l'hydrocyclone (3) et/ou les pompes (5, 10) ou les têtes de celles-ci et/ou les conduites (4, 7, 9, 12, 18) sont réalisées sous la forme de composants à usage unique.

8. Procédé pour l'évacuation continue d'une solution pauvre en particules hors d'un bioréacteur (2) au moyen d'une séparation de particules dans un hydrocyclone (3), une solution pauvre en particules étant prélevée hors du bioréacteur (2) via une conduite d'alimentation (4) contenant une première pompe (5) intercalée, étant acheminée vers l'hydrocyclone (3) via une admission (6, 16) et étant divisée en un flux supérieur pauvre en particules et un flux inférieur plus riche en particules, le flux inférieur pouvant être renvoyé dans le bioréacteur (2) via une conduite de retour (7) et le flux supérieur étant évacué, **caractérisé en ce qu'**une première partie du flux inférieur est acheminée à nouveau vers l'admission (6, 16) de l'hydrocyclone (3) via une conduite de dérivation (9, 18) contenant une deuxième pompe (10) intercalée, tandis que la deuxième partie du flux inférieur est renvoyée dans le bioréacteur (2).

9. Procédé selon la revendication 8, **caractérisé en ce qu'**un milieu contenant des cellules est acheminé sous forme de solution depuis le bioréacteur (2) dans l'hydrocyclone (3) et est divisé en un flux supérieur pauvre en cellules et un flux inférieur riche en cellules.

10. Procédé selon la revendication 8 ou 9, **caractérisé en ce qu'**une différence de pression, nécessaire dans l'hydrocyclone (3), est réglée par l'intermédiaire de la deuxième pompe (10).

11. Procédé selon l'une quelconque des revendications 8 à 10, **caractérisé en ce que** le taux de dilution du bioréacteur (2) est réglé par l'intermédiaire de la première pompe (5).

12. Procédé selon l'une quelconque des revendications 8 à 11, **caractérisé en ce que** le flux supérieur pauvre en cellules est acheminé vers un filtre (17) et est filtré par celui-ci.
